# EUROPEAN PATENT SPECIFICATION

(11) **EP 2 004 137 B1**
(45) Date of publication and mention of the grant of the patent: **04.01.2012**
(21) Application number: 07732393.9
(22) Date of filing: 12.04.2007
(51) Int. Cl.: A61K 8/85, C08G 63/20

(54) **STRUCTURANTS FOR OIL PHASES**
STRUKTURANTIEN FÜR ÖLPHASEN
STRUCTURANTS POUR PHASES HUILEUSES

(30) Priority: 13.04.2006 GB 0607500
(43) Date of publication of application: 24.12.2008
(73) Proprietor: CRODA INTERNATIONAL PLC, Goole North Humberside DN14 9AA (GB)
(72) Inventor: BEVINAKATTI, Hanamanthsa Shankarsa, Cleveland TS17 5GD (GB); WAITE, Alan Geoffrey, County Durham DL1 1DH (GB)
(74) Representative: Humphries, Martyn
(86) International application number: PCT/GB2007/001350
(87) International publication number: WO 2007/119047

(56) References cited:
- US-A- 5 055 548
- US-A1- 2006 019 848
- DATABASE WPI Week 199420 Derwent Publications Ltd., London, GB; AN 1994-164081 XP002441183 & JP 06 107866 A (HARIMA KASEI KK) 19 April 1994 (1994-04-19)
- DATABASE WPI Week 198535 Derwent Publications Ltd., London, GB; AN 1985-215110 XP002441184 & RO 85 847 A (CENT PROTECT ANTICOROZIV) 30 January 1985 (1985-01-30)

## Description

This invention relates to structurants for oil phases, particularly to oil structurants which are oligoesters reaction products of polyols, dimer dicarboxylic acids and long chain monocarboxylic fatty acids, to oil phases including them and to the use of such structured oil phases particularly in personal care products such as cosmetics.

Oils possess highly desirable cosmetic characteristics, such as cleansing, make-up removal, and emolliency, but their fluid form makes them inconvenient to use and/or makes their application difficult and sometimes unpleasant. Such disadvantages can be reduced by using the oil in the form of a structured or particularly a thickened composition, such as a cream or a gel or in the form of an emulsion, particularly a water in oil emulsion, where thickening is only needed for the continuous oily phase. Thickening the oily phase is also done to prepare cosmetic gels, particularly anhydrous gels which are useful, particularly where substances in the composition are sensitive to moisture and/or to oxygen.

Materials that are available for oil structuring, thickening or gelling include waxes, silicas, such as fumed silica, hydrophobically modified clays such as bentonites, fatty acid metal salts, such as aluminium magnesium hydroxide stearate, hydroxyfatty acid esters, such as trihydroxystearin, or oligosaccharide ester derivatives such as dextrin palmitate. While they can be effective structurants, gelling agents and thickeners, these materials do have disadvantages. For example, waxes can give the end products an undesirable skin feel and may reduce the gloss of products, very disadvantageous for lipsticks, and other personal care products; hydrophobically modified clays and aluminium magnesium hydroxide stearate structured oils do not spontaneously emulsify on dilution with water, and hydrophobically modified clays have high mixing energy requirements, trihydroxystearin and high molecular weight polymers generally require the use of high processing temperatures e.g. up to 90°C, in making structured oils, silicas have low bulk density, and dextrin palmitates are very expensive.

US 2006-019848 discloses a high gloss, non-feathering topical composition comprising at least one water-insoluble, fatty alcohol-soluble polysaccharide polymer and a liquid polymeric polyol ester.

The present invention is based on our finding that ester products obtainable by reaction of polyols, dimer dicarboxylic acids and long chain monocarboxylic fatty acids and in particular such products that have multiple pendent fatty acid groups usually also with free hydroxyl groups, can be very effective structurants in oil phase systems.

The present invention accordingly provides a polyester oil structurant compound which is obtainable by the reaction of a dimer dicarboxylic acid, derived from oleic, linoleic and/or linolenic acids, a polyol having at least 3 hydroxyl groups with an average of from 1 to 2 primary hydroxyl groups and at least one, particularly 1 to 4, secondary hydroxyl groups(s) and a C₂₀ to C₃₀, particularly a C₂₀ to C₂₄, monocarboxylic fatty acid.

The compounds of the present invention are designed for use as oil structurants, particularly thickeners and/or gellants, and the invention accordingly includes a structured oil system which includes as an oil phase structurant a compound which is polyester oil structurant compound which is obtainable by the reaction of a dimer dicarboxylic acid, derived from oleic, linoleic and/or linolenic acids, a polyol having at least 3 hydroxyl groups with an average of from 1 to 2 primary hydroxyl groups and at least one, particularly 1 to 4, secondary hydroxyl groups(s) and a C₂₀ to C₃₀, particularly a C₂₀ to C₂₄, monocarboxylic fatty acid.

The compounds of the invention find particular use as structurants in oil based or oil containing personal care formulations and the invention accordingly includes a personal care product or formulation which includes a thickened oil system which includes as a structurant a polyester compound which is obtainable by the reaction of a dimer dicarboxylic acid, a polyol, which desirably has an average of form 1 to 2 primary hydroxyl groups and at least one, particularly 1 to 4, secondary hydroxyl group(s) and particularly is selected from glycerol, sorbitol, sorbitan and mixtures or combinations of these, and a C₁₆ to C₃₀, particularly a C₁₈ to C₂₄, more particularly a C₂₀ to C₂₄, monocarboxylic fatty acid.

Additionally the invention includes the use of the compounds of the invention as oil structurants, in particular in personal care products or formulations.

In referring to compounds of and used in this invention as "polyesters" or "oligoesters", we are referring to the multiple ester linkages in the compounds - derived from reaction between the polyol and the di- and mono-carboxylic acids. They do not necessarily imply that the compounds have polyester chains - of alternating dicarboxylic acid and polyol residues, although such chains are a desirable feature of many compounds of and used in the invention.

The term "structurant" refers to the provision of effects ranging from increasing the viscosity (viscosifying or thickening) to gelling the oil (creating a three dimensional structure at the molecular level which "traps" the continuous phase oil) and includes the possibility of generating liquid crystal like phases in the oil, all of which can enhance the stability of dispersed phases in the oil.

The polyol used as a starting material in making the polyesters of the invention groups have at least 3 and typically up to 8 hydroxyl groups and has an average of from 1 to 2 primary hydroxyl groups and at least one, particularly 1 to 4, secondary hydroxyl group(s). They can be considered as being of the formula (I): R¹-(OH)ₙ, where n is from 3 to 8 and particularly from 3 to 6. The group R¹ is desirably aliphatic and is typically hydrocarbyl, usually saturated, having from 3 to 10 and particularly 3 to 8, and especially 3 to 6, carbon atoms and will usually be linear though it may include branching. In the polyol (I) there will generally be one or two primary hydroxyl groups (on the polyol terminal carbon atoms) and at least one and commonly n-2 secondary hydroxyl groups. Desirably, the polyol (I) is of the formula (Ia): HOH₂C-(CHOH)p-CH₂OH where p is from 1 to 6, more usually from 1 to 4. Suitable polyols include glycerol, C₄ polyols such as threitol and erythritol, C₅ polyols such as inositol, arabitol and xylitol and C₆ polyols such as sorbitol, and derived materials such as sorbitan. The C₄ to C₆ polyols are commonly the reduced or hydrogenated forms of corresponding tetrose, pentose and hexose sugars. Particularly desirably the polyol is glycerol, and especially sorbitol and sorbitan (usually derived *in situ* from sorbitol) or a mixture or combination of these.

A further group of useful polyols are the oligocondensation products of polyols such as glycerol and pentaerythritol. Oligoglycerols, commonly called polyglycerols, are typically of the formula (Ib): H[-O-Gly-]ₙ-OH (Ib) where each Gly is independently the residue of a molecule of glycerol after removal of two hydroxyl groups; and n is (an average of) from 2 to 10. Generally most of the groups Gly will be of the formula: -CH₂-CHOH-CH₂-, the residues after etherification reaction at the primary hydroxyl groups although groups such as -CH-(CH₂OH)-CH₂- and -CH₂-CH(CH₂OH)-, the residues after etherification at one primary and the secondary hydroxyl group, may also be present. Examples of oligoglycerols include diglycerol, triglycerol, tetraglycerol, pentaglycerol, hexaglycerol, heptaglycerol, nonaglycerol, decaglycerol and mixtures of these. Particularly useful polyglycerols are those of the formula (I) where n is particulalry from 2 to 7, more particularly from 2 to 5 and especially 2, 3 or 4, or mixtures of oligoglycerols in these ranges.

Oligomers of pentaerythritol are generally of the formula (Ib): H[-OCH₂C(CH₂OH)₂CH₂-]ₘOH (Ib) where m is (an average of) from 1.5 to 5, particularly 2 to 4.

It is possible to include relatively small proportions of residues derived from diols e.g. as derived from ethylene, diethylene, triethylene or propylene glycols or isosorbide (derived by dianhydridisation of sorbitol). The inclusion of such diol residues may enable adjustment of properties of the polymers, however, the proportion of such residues will generally be low, typically an average of not more than 20 mol%, more usually not more than 15 mol%, and desirably not more than 10 mol% e.g. from 1 to 10 mol%, and particularly from 1 to 5 mol% of the polyol residues in the compounds.

Especially where the polyol has four or more carbon atoms and four or more hydroxyl groups usually two primary hydroxyls and 2 or more secondary hydroxyls, and in particular where the polyol is of the formula (Ia) with p being 3 or 4, it may be susceptible to react by an intramolecular etherification (anhydridisation) reaction to form cyclic ethers. For example sorbitol can form sorbitan cyclic ethers which may react further to form the dicyclic diether iso-sorbide, reducing the number of hydroxyl groups available for esterification. When sorbitan residues are desired in the product, it will usually be done by, in effect, *in situ* formation of the sorbitan; and correspondingly it is likely that some sorbitol will be converted into sorbitan when trying to make sorbitol esters. Although anhydridisation will occur its extent may be controlled (at least to a limited extent) by selection of the reaction conditions e.g. use of acidic catalysts, particularly if linked with higher reaction temperatures, will lead to a greater degree of anhydridisation than the use of alkaline catalysts (and lower reaction temperatures will lead to a lower degree of anhydrisation).

The dimer dicarboxylic acid used as a starting material in making the polyesters of the invention is or includes residues based on fatty acid dimer residues. Fatty acid dimers (commonly referred to simply as "dimer acids") are the well known mainly dimeric oligomerisation products derived from unsaturated fatty acids (industrially principally oleic, linoleic and/or linolenic acids), typically thermally polymerised, usually using acid catalysts e.g. acidic sites on clay. Generally they have average molecular weights corresponding to approximately two molecules of the starting fatty acid, so dimerised oleic acid has an average molecular weight corresponding to a nominally C₃₆ diacid, though as described below, commercial grades will typically includes also C₁₈ ("monomer") and C₅₄ ("trimer") components. As manufactured, dimer acids have unsaturation but this may be reduced (hydrogenated) in making starting materials for the polymers used in this invention.

Dimer acids are commercially made as distillation fractions from the polymerisation reaction described above. Typically, they will include small proportions of monocarboxylic materials. The proportion of monocarboxylic is desirably kept relatively low as monocarboxylic compounds will tend to act as chain stoppers in the polyesters thus reducing the effective molecular weight achievable. Also, as the monmeric acids will generally be branched and may be unsaturated, they may have a detrimental effect on the oil structurant properties of the oligomeric esters. Generally the proportion of residues from such monofunctional carboxylic acid in the polymer will not be more than about 5 mol%, more usually not more than about 3 mol%, and desirably not more than about 2 mol%, of the total dimer acid used.

The oligomerisation reaction that is used to make dimer acids will also generally give rise to trimeric products (sometimes called trimer "acid") and dimer acids typically include at least some trimer acid. Tricarboxylic compounds, such as trimer acid, will provide sites for chain branching and may lead to crosslinked polymers. This may reduce to effectiveness of the polymers as structurants and for this reason may be undesirable, although the effect may be used to adjust the structuring properties of the polymers. The proportion of residues of such trifunctional carboxylic compounds in the polymers used in the invention may be up to about 30 mol%, more usually not more than about 25 mol%, of the total dimer acid residues used. Amounts from 1 to 25 mol%, more usually from 2 to 20 mol%, of the total dimer acid residues used will be typical. We have not found it necessary to use purified dimer acid to make satisfactory structurants.

The dimer acids can be used as the free acid or as suitable reactive derivatives, particularly lower e.g. C₁ to C₄ and particularly methyl, alkyl esters (usually diesters).

Other dicarboxylic acids may be included if desired e.g. to modify the structurant properties of the compounds such diacids can be C₄ to C₁₂, e.g. C₄ to C₁₀, dicarboxylic acids and will usually be aliphatic compounds. Typically, such dicarboxylic acids are of the formula: HOOC-R²-COOH, where R² is a C₂ to C₈ hydrocarbyl group which can be saturated or unsaturated, linear or branched and can be aromatic e.g. a phenyl ring (thus giving a phthalic, terephthalic or iso-phthalic dicarboxylic acid) or and desirably aliphatic, typically an alkylene or alkenylene group, and may be cyclic though it is desirably open chain. Commonly R² is a group: -(CH₂)ₘ-, where m is from 2 to 8. Because mixtures of different dicarboxylic acids (or reactive derivatives) may be used to make materials used in practice, m may appear to be non integral, because it will be an average. Suitable reactive derivatives of the dicarboxylic acids include lower e.g. C₁ to C₄ and particularly methyl, alkyl esters (usually diesters) and anhydrides, particularly cyclic anhydrides such as succinic, maleic and phthalic anhydrides. The "other" dicarboxylic acid may include higher functional carboxylic acids e.g. tricarboxylic acids, such as trimellitic acid in addition to tricarboxylic acid included in the dimer. If included the proportion of dicarboxylic acid that is other than dimer acid will typically not be more than about 70 mol%, more usually not more than about 60 mol%, and desirably not more than about 50 mol%, of the total dicarboxylic acid (dimer acid plus other dicarboxylic acid) used. Correspondingly, the proportion of dimer acid will typically be at least about 30 mol%, more usually at least about 40 mol%, and desirably at least about 50 mol%, of the total dicarboxylic acid (dimer acid plus other dicarboxylic acid) used. When other dicarboxylic acids are used the proportion used will generally be from 2 to 70, more usually from 5 to 60 and typically from 10 to 50, mol% of the total dicarboxylic acid. Considering that the dimer diacids have substantially higher molecular weights than the "other" dicarboxylic acids, these ranges broadly correspond to about 0.5 to 35, 1.1 to 25 and 2.3 to 20,% by weight of the total dicarboxylic acid.

The monocarboxylic acid used as a starting material in making the polyesters of the invention are C₂₀ to C₃₀ and desirably C₂₀ to C₂₄ hydrocarbyl, particularly aliphatic and especially saturated, linear aliphatic fatty acids. Typically they are of the formula (III): R⁵CO₂H, where R⁵ is a C₁₉ to C₂₃ aliphatic hydrocarbyl, particularly alkyl, group. Reactive derivatives of monocarboxylic acids that can be used in the synthetic reaction include lower e.g. C₁ to C₄ and particularly methyl, alkyl esters.

The length of the monocarboxylic fatty acid chains appears to be directly related to the structuring effectiveness of compounds of and used in the invention - the use of shorter chain fatty acids than C₁₆ appears to give little if any structuring e.g. thickening, viscosifying or gelling effect (at least at ambient temperature) and we have found that longer chains such as C₂₀ or longer chains can give more effective structurants. Thus, a desired monocarboxylic acid is especially, behenic acid. The presence of branching or unsaturation in the chains of the monocarboxylic acids is generally undesirable as it makes products based on such acids much less effective structuring agents. The proportions of branched or unsaturated monocarboxylic acids will generally be low, typically an average of not more than 10 mol%, more usually not more than 5 mol%, and desirably not more than 2 mol% e.g. from 1 to 5 mol%, of the total moncarboxylic fatty acid residues in the compounds.

Mixtures of monocarboxylic acids may be used if desired and it may be advantageous in permitting the properties of the products to be adjusted. Using combinations of long chain monocarboxylic acids will generally give products whose properties are intermediate between the properties of the respective products made wholly with the respective fatty acids. Including small proportions of short chain (particularly shorter than 16 carbon atoms) or branched or unsaturated monocarboxylic acids (of any chain length) will tend to make the products less effective as gellant type structurants. However, including relatively small proportions of such shorter chain monocarboxylic acids or of branched or unsaturated monocarboxylic acids may be beneficial in enabling the properties of the structurant to be modified e.g. to improve sensorial properties such as skin feel. The proportions of short chain monocarboxylic acids will generally be relatively modest, typically an average of not more than 30 mol%, more usually not more than 25 mol%, and desirably not more than 20 mol% e.g. from 1 to 20 mol%, and particularly from 5 to 15 mol% of the total moncarboxylic fatty acid residues in the compounds.

Significant thickening can be achieved with even relatively small polyester molecules and the compounds of and used in the invention typically have a number average molecular weight (in Daltons) of from 1000 to 10000, more usually from 1200 to 7000, and particularly from 1300 to 6000 and a corresponding weight average molecular weight of from 2500 to 20000, more usually from 2500 to 12000, and particularly from 2500 to 10000. (Based on molecular weights measured by gel permeation chromatography against polystyrene standards.) Such molecular weight values correspond to chain lengths derived from the nominal polymerisation esterification of the polyol and the dimer dicarboxylic acid (which may also include other dicarboxylic acid) of from about 1 to about 20, more usually from about 1 to about 10 and particularly from about 1 to about 7.5, repeat units (based on a hydroxy/carboxy ended polyol-dicarboxylic acid ester unit). Of course, the number of "repeat units" and molecular weight are average values and may thus be non-integral.

The extent to which the total available hydroxyl groups in the reaction components used in making the products of the invention are esterified can have a significant effect on the efficiency of the compounds of and used in the present invention as structurants. Generally a minimum of 40%, more usually at least 50% and desirably at least 60% of the total polyol hydroxyl groups will be esterified. At lower levels of esterification, the proportion of free hydroxyl groups is sufficiently high to significantly reduce the oil solubility of the oligoesters and thus to have a detrimental effect on the thickening performance of the compounds. Clearly the maximum number of such ester residues will depend on the number of hydroxyl groups in the original polyol. However, in practice it is difficult to achieve complete reaction so the level of esterification is generally not more than about 90% and commonly not more than about 80% of the hydroxyl groups in the original polyol. (In reckoning the number of hydroxyl groups in the original polyol, any that react to form ethers under the reaction conditions e.g. as in forming sorbitol from sorbitan, are not included.) Within these ranges, the proportion of free hydroxyl groups may be used to modulate or moderate the thickening effects of the compounds.

Overall, it is desirable that the oligomers have an excess of hydroxyl groups over the carboxyl groups. The molar ratio of OH:COOH groups (based on the amounts of these functional groups in the starting materials) is desirably at least 1.3, more usually at least 1.5 and can be up to 2.8, though more usually up to about 2.5.

In order to generate a preponderance of hydroxyl (over carboxyl) ends in the product oligoester, the polyol will generally be used in molar excess over the dimer (and any other) dicarboxylic acid, most commonly at a molar ratio of polyol : diacid of from 1.1 to 2, more usually from 1.25 to 2. The amount of the monocarboxylic acid used will depend on the number of hydroxyl groups in the polyol and the proportion of hydroxyls that it is desired to esterify in the product. Generally the proportion used will be from 1 to 2.5, more usually from 1 to 2.2, and especially from 1.2 to 2, moles monocarboxylic acid per mole of polyol. Accordingly, the ratios of the polyol, dimer (and any other) dicarboxylic acid and monocarboxylic acid used will generally be in the range 1 : 0.5 to 0.95 : 1 to 2.5, more usually 1 : 0.5 to 0.85 : 1 to 2.2, and desirably 1 : 0.6 to 0.85 : 1.2 to 2. The products of the invention typically have a hydroxyl number (OH no) of from 15 to 350, more usually from 25 to 300.

The compounds of and used in the invention are oligo- or poly-esters of the polyol, dicarboxylic, including dimer, acid and monocarboxylic acid, but their exact molecular structure is unclear. From molecular weight measurements using gel permeation chromatography it appears that the polyol and dicarboxylic, including dimer, acid are linked to form an oligo- or poly-meric skeleton and that the monocarboxylic acid is esterified to some or all of the remaining polyol hydroxyl groups. It is probable that the compounds are mixtures of different molecules and are likely to include some relatively straight linear chains e.g. as from reaction between the dicarboxylic, including dimer, acid and α,ω-primary hydroxyl groups as in glycerol or sorbitol, some "kinked" chains such as arising from reaction between the dicarboxylic, including dimer, acid and non terminal, particularly secondary, hydroxyl groups, and some branched or even crosslinked chains or groups.

The compounds of and used in the invention are mainly OH ended and the proportion of free carboxylic acid groups is small, as reflected in the measured acid values which typically corresponding to a significantly higher equivalent weight for the carboxylic acid content than the measured molecular weight. Typically acid values will commonly be less than 10, e.g. less than 5, mg(KOH).g⁻¹ though where the polyol is susceptible to anhydridisation e.g. sorbitol, and the product is desired to have low levels of anhydridisation, less driving conditions may be used in the esterification and this may result in somewhat higher acid values e.g. up to 20 mg(KOH).g⁻¹. Reflecting that the products of and used in the invention are mainly OH ended (rather than carboxyl ended) the OH no will usually be significantly higher than the acid value e.g. at least 1.5 times the acid value (both in mg(KOH).g⁻¹).

The compounds of and used in the invention can be made by a generally conventional esterification using a polyol, the dimer and possibly other dicarboxylic acid (or reactive derivative(s)) and a monocarboxylic acid (or reactive derivative) as starting materials. We have successfully made the compounds of and used in the invention by a direct one stage route in which all three components: the polyol, the dicarboxylic acid(s) (or reactive derivative(s)) and the monocarboxylic acid (or reactive derivative) are mixed and reacted together under (trans-) esterification conditions, particularly at elevated temperatures and in the presence of a catalyst.

The reaction conditions will typically involve using a reaction temperatures of from 150 to 250°C, and particularly 170 to 240°C. Where free acids are used as reagents in direct esterification, the reaction may be carried out under atmospheric pressure or under moderate vacuum e.g. at pressures from 50 to 250 mBar, particularly about 100mBar gauge to facilitate removal of water of reaction, and trans-esterification reactions using lower alkyl esters will usually be carried out at ambient pressure.

Suitable catalysts will depend on the actual starting materials and the desired product. For direct esterifications typical catalysts include basic catalysts such as alkali metal hydroxides or carbonates, such as sodium or potassium hydroxide or carbonate, particularly potassium carbonate; or acidic catalysts, such as sulphonic acids e.g. p-toluene sulphonic acid or phosphorus oxyacids such as phosphoric acid, or, particularly if it is desirable to avoid colour forming oxidation reactions, especially with starting polyols such as sorbitol, phosphorous acid, and catalysts combining phosphoric and/or phosphorous acid with alkali, typically at a molar ratio of from 1:1 to 1:3; and for trans-esterifications typical catalysts include relatively mild alkali metal base such as carbonates, particularly potassium carbonate, titanate esters, such as tetrabutyl titanate.

The invention further includes a method of making a structurant compound of the invention which comprises reacting a polyol (or reactive derivative), a dicarboxylic, including dimer, acid (or reactive derivative) and a fatty monocarboxylic acid (or reactive derivative) together under esterification conditions to form a fatty polyester structurant derivative.

A wide range of oils can be structured using the compounds of the invention and the best such compounds will provide structuring in a wide range of oils (rather than a relatively narrow range for each structuring compound). The range of oil polarity for which structuring can be provided is wide ranging from non-polar oils such as paraffinic oils to alkoxylate oils. One way of expressing this range of polarity is to use a numeric solubility parameter. We have found that Hansen and Beerbower solubility δ^{t} parameter combining dispersive (van der Waals), polar (Coulombic) and hydrogen bonding component (see the CRC Handbook of Solubility Parameters and Other Cohesion Parameters pp 85 to 87) provide good correspondence with the polarity as reflected in the performance of the oils that we have investigated. The numerical values of solubility parameter given below are Hansen and Beerbower δ^{t} values abbreviated as "HBSP" values. Generally structurants of and used in this invention can provide structure in oils with HBSP values ranging from 15 (very non-polar) to 25 (highly polar) particularly from 15 to 22.

Typical oils that can be structured using compounds of the invention include:
fatty alcohol polyalkoxylate, particularly propoxylates such as the alkoxylates of C₁₂ to C₂₀ fatty, particularly C₁₄, C₁₆ and C₁₈ fatty alcohols which can be linear e.g. as in palmitic and stearic acids, or branched e.g. as in isostearyl alcohol (in practice a product typically derived from dimer acid manufacture which contains a mixture of mainly branched C₁₄ to C₂₂ alcohols averaging about C₁₈), with from 3 to 25 particularly from 7 to 20 alkoxylate alkoxylate, especially ethoxylate, propoxylate or mixtures of ethoxylate and propoxylate, units e.g. the stearyl alcohol 15-polypropoxylate available from Uniqema under the tradename Arlamol E (HBSP 20.8);
ester oils particularly those based on C₂ to C₂₂ linear, branched or unsaturated fatty acids and linear, branched or unsaturated fatty alcohols, and typically esters derived from monocarboxylic acid(s) with monohydric alcohol(s); di- or tri-carboxylic acid(s) with monohydroxy alcohol(s) acid with monohydric alcohol(s); or di- or poly-hydric alcohol(s) with monocarboxylic acid(s), e.g. the ester oil available from Uniqema under the tradename Estol 3609 (HBSP 20.4), the isopropyl isostearate oil available from Uniqema under the tradename Prisorine 2021 (HBSP 17.7) and the methyl oleate oil available from Uniqema under the tradename Priolube 1400 (HBSP 17.9);
aromatic ester oils, particularly esters if benzoic acid and C₈ to C₁₈ monohydric alcohol(s) e.g. the C₁₂ to C₁₅ benzoate oil from Finetex under the tradename Finsolve TN (HBSP 19.1);
branched liquid fatty alcohols, particularly Guerbet alcohols e.g. octyldodecanol or isostearyl alcohol (see above) e.g. the isostearyl alcohol available from Uniqema under the tradename Prisorine 3515 (HBSP 17.9);
branched liquid fatty acids, particularly isostearic acid and dimer acid (dimerised fatty acids, particularly oleic and/or linoleic acids), such as dilinoleic acid (HBSP 17.8); and
paraffinic oils, especially branched paraffinic oils e.g. the iso-paraffinic oil available from Uniqema under the tradename Arlamol HD (HBSP 15.6);
silicone oils, which may be volatile or non-volatile, particularly dimethylpolysiloxane oils (dimethicone oils), including cyclomethicone oils, and silicone oils with non-methyl substituents as in phenyl trimethicone.
The oils, particularly the above oils can be used as mixtures of two or more different types of oils e.g. mixtures of silicone oils and ester oils.

The amount of the structurant included in oil based formulations will depend on the desired effect, but will usually be in the range from 0.1 to 10% by weight, more usually from 0.2 to 7%, and particularly from 0.5 to 5% by weight of the formulation.

The compounds of and used in this invention may be used alone or, if desired in combination with other structurants, particularly to ensure that the desired structuring effect it achieved the entire temperature range required for a particular product. when used with other structurants, the proportion of structurant of the invention will generally be from 25 to 95%, more usually from 40 to 80%, by weight of the total structurant used. The total amount of structurant when mixtures are used will generally be within the ranges given above for the compounds of the invention.

The structurants will generally be incorporated into the oil based formulations by dissolving the structurant in the oil, usually at moderately elevated temperature typically from 50 to 90°C, more usually from 60 to 85°C, commonly at about 80°C, and then cooling the mixture or allowing the mixture to cool to ambient temperature. The structuring effects become apparent on cooling.

The compounds of and used in the invention are useful as structurants in oil based systems, in particular in personal care formulations. The desirable effects that they will be used to provide may include: increased viscosity ranging from modest thickening to true gelling which can be useful in making anhydrous gels which can protect moisture and/or to oxygen sensitive formulation components; modifying the rheological profile, to alter formulation delivery or spreading, or to provide improved colour or UV protection; improved surface adhesion, giving better wear or water resistance or non-transfer properties; modified tactile sensory properties giving reduced perceived oiliness or to create an impression of softness; or improved formulation stability by reducing or preventing separation, sedimentation or syneresis. The physical form of such personal care formulations includes dispersions of cosmetic materials e.g. pigments, sunscreen components or other active materials in oil based formulations; in water in oil emulsions e.g. skin creams; or in oil in water formulations to reduce perceived oiliness after application particularly after the water continuous phase evaporates from or is absorbed into the skin. The types of personal care product to which this technology is applicable include decorative cosmetics such as lipstick, mascara, cosmetic foundation and cream powders; antiperspirant and deodorant products, particularly sticks and gels; baby oils (particularly based on paraffinic oils) and other skin care oil mixtures including hair, massage, make up remover and cleansing products; shampoos, particularly where a relatively viscous carrier material is desired; and sun care products, particularly sunscreens e.g. those based in whole or in part on ultrafine pigments such as titanium dioxide or zinc oxide or similar materials. The amount of the structurant included in such personal care formulations will generally be within the ranges given above and other structurants may be included if desired also as described above.

Other end uses of the compounds of the invention include as structurants in oil based crop protection formulations, particularly so-called oil flowable formulations; and as structurants in greases.

The following examples illustrate the invention. All parts and percentages are molar unless otherwise stated.

### Materials

- P1: sorbitol
- DA1: oleic dimer acid - Pripol 1017 ex Uniqema
- DA2: hydrogenated oleic dimer acid - Pripol 1006 ex Uniqema
- DA3: 1:1 molar mixture of sebacic acid and DA1
- FA1: palmitic acid/stearic acid blend 1:1 molar - Pristerene 9559 ex Uniqema
- FA2: behenic acid - Prifrac 2987 ex Uniqema
- Cat1: potassium carbonate
- Cat2: a mixture of H₃PO₃ and NaOH in a molar ratio of 1.59:4.0
- Oil1: stearyl alcohol 15-polypropoxylate - Arlamol E ex Uniqema
- Oil2: glycerol tris-2-ethylhexanoate ester oil - Estol 3609 ex Uniqema
- Oil3: C12/15 benzoate ester mixture - Finsolv TN ex Finetex
- Oil4: isostearyl alcohol - Prisorine 3515 ex Uniqema
- Oil5: isopropyl isostearate - Prisorine 2021 ex Uniqema
- Oil6: isohexadecane emollient oil - Arlamol HD ex Uniqema
- Oil7: methyl oleate - Priolube 1400 ex Uniqema

### Test methods

### Acid Value - was measured using the ASTM D1980-87 method and the results are quoted as "AV" in mg(KOH).g⁻¹.

### Hydroxyl No - was measured using the ASTM D1957-88 method and the results are quoted as Free hydroxyl ("Free OH" no is OH - AV) "OH" in mg(KOH).g⁻¹

Viscosity was measured on a solution of structurant generally at a concentration of 5 wt% (a few were done at 10 wt%) in the test oil using a Brookfield DV1+ viscometer generally at 10 rpm (a few were measured at 5 rpm as indicted with the data) using T bar S95, with the measurement being made 1 minute after starting rotating the T bar, and the results are quoted in Pa.s.

Molecular Weight was measured by gel permeation chromatography on a Viscotek Evolution GPC system using a mixed gel column and THF eluent against polystyrene standards. The results enabled both number and weight average molecular weights to be calculated.

### Synthesis Examples

### Synthesis Example SE1 Comparative) - Poly(sorbitol dimerate) stearate/palmitate

Anhydrous polyol P1 (47.3 g; 0.26 mol), dimer acid DA1 (88.0 g; 0.16 mol), monocarboxylic fatty acid FA1 (105.3 g; 0.39 mol) and catalyst Cat1 2.61 g (7.5 mol% based on polyol) were charged to a 500 ml round bottomed flask fitted with a propeller stirrer, side-arm water condenser and collection flask, vacuum pump, nitrogen sparge and thermometer (thermocouple) and an isomantle. The mixture was heated under stirring (300 rpm) to 170°C with a nitrogen sparge under a vacuum of 100 mbar. The reaction was stopped when the acid value was < 5 mg(KOH).g⁻¹ (after 7 hrs) and the product discharged. The measured molecular weight was Mn 2220; Mw 6390.

### Synthesis Example_SE2 - Poly(sorbitol dimerate) behenate

Anhydrous polyol P1 (40.2 g; 0.22 mol), dimer acid DA2 (74.3g; 0.13 mol), monocarboxylic fatty acid FA2 (135.4 g; 0.40 mol), catalyst Cat2 0.28g (1.59 mol % of phosphorous acid based on polyol) were charged to a 500ml round bottomed flask fitted with a propeller stirrer, side-arm water condenser and collection flask, nitrogen sparge and thermometer (thermocouple) and an isomantle. The mixture was heated under stirring (300 rpm) to 240°C with a nitrogen sparge. The reaction was stopped when the acid value was < 5 mg(KOH).g⁻¹ (after 7 to 8 hrs) and the product discharged. The measured molecular weight was Mn 2400; Mw 6370.

Further oligo-(sorbitol dimerester) esters were made by the general methods set out in Syntheses Examples SE1 and SE2 but making appropriate changes to the material proportions or conditions. The products made in the Synthesis Examples and the reaction conditions used are summarised in Table 1 below.

**Table 1**

| Ex No | Polyol | | Dimer acid | | Mono acid | | Catalyst | | Temp | Press | Time | AV | OH |
|---|---|---|---|---|---|---|---|---|---|---|---|---|---|
| | Type | mol | Type | mol | Type | mol | Type | mol | (°C) | (mbar) | (hrs) | | no |
| SE1 | P1 | 1 | DA1 | 0.6 | FA1 | 1.5 | Cat1 | 7.5 | 170 | 100 | 7 | 3.7 | 294 |
| SE2 | P1 | 1 | DA2 | 0.6 | FA2 | 1.8 | Cat2 | 1.59 | 240 | atm | 7.5 | 4.5 | 29 |
| SE3 | P1 | 1 | DA1 | 0.6 | FA2 | 1.5 | Cat1 | 7.5 | 170 | 100 | 12 | 6.3 | 159 |
| SE4 | P1 | 1 | DA2 | 0.8 | FA2 | 2.0 | Cat2 | 1.59 | 240 | atm | 4.5 | 10.7 | 47 |
| SE5 | P1 | 1 | DA3 | 0.6 | FA2 | 1.5 | Cat1 | 7.5 | 170 | 100 | 9 | 11.8 | 179 |
| SE6 | P1 | 1 | DA2 | 0.5 | FA2 | 1.5 | Cat1 | 7.5 | 200 | atm | 2 | 18.0 | 188 |
| SE7 | P1 | 1 | DA2 | 0.5 | FA2 | 1.5 | Cat1 | 7.5 | 200 | atm | 3 | 10.0 | 173 |

### Comparative Application Example AE1 and Application Examples AE2 to AE5

Test formulations were made by dissolving the polymers of Synthesis Examples SE1 to SE7 in various oils by heating a mixture of oil and structurant to 80°C under moderate stirring and allowing the mixture to cool to ambient temperature once the structurant had dissolved. In Examples AE2, AE4 and AE5 5% by weight polymer and in Examples AE1 and AE3 10% by weight polymer, both on the combined polymer and oil was used. The viscosity of the oils was measured the day following make up of the structured oil and the results are set out in Table 2 below.

**Table 2**

| Ex No | Viscosity (Pa.s) | | | | | | |
|---|---|---|---|---|---|---|---|
| | Oil1 | Oil2 | Oil3 | Oil4 | Oil5 | Oil6 | Oil7 |
| 1 | 36.7 | 47.5 | (1) | (1) | (1) | 15.8 | - |
| 2 | 98 | 55 | 0.018 | 48 | 0.021 | 0.025 | - |
| 3 | 52.5 | 49.8 | 29.5 | 113 | 11.6 | 8.3 | - |
| 4 | 98.5 | 35 | 0.020 | 10.5 | 0.020 | 1.5 | - |
| 5 | 0.080 | 4.2 | 50 | (1) | 2.5 | 16.5 | - |
| 6 | 84 | - | - | 13 | - | 16.5 | 127 |
| 7 | 196 | - | - | 21 | - | 2.5 | 105 |

| | | | | | | | |
|---|---|---|---|---|---|---|---|
| (1) product too insoluble to make up test solution | | | | | | | |

## Claims

1. A polyester oil structurant compound which is obtainable by the reaction of a dimer dicarboxylic acid, derived from oleic, linoleic and/or linolenic acids, a polyol having at least 3 hydroxyl groups with an average of from 1 to 2 primary hydroxyl groups and at least one, particularly 1 to 4, secondary hydroxyl groups(s) and a C₂₀ to C₃₀, particularly a C₂₀ to C₂₄, monocarboxylic fatty acid.

2. A polyester oil structurant compound as claimed in claim 1 wherein the polyol is glycerol, sorbitol, sorbitan or a mixture or combination of these.

3. A polyester oil structurant compound as claimed in either claim 1 or claim 2 wherein the dimer dicarboxylic acid is based on oleic acid dimer.

4. A polyester oil structurant compound as claimed in any one of claims 1 to 3 wherein the dicarboxylic acid includes in addition to the dimer dicarboxylic acid a dicarboxylic acid is of the formula HOOC-R²-COOH, where R² is a C₂ to C₈ hydrocarbyl group.

5. A polyester oil structurant compound as claimed in claim 4 wherein R² is a group -(CM₂)ₘ-, where m is from 2 to 8.

6. A polyester oil structurant compound as claimed in claim 5 wherein the monocarboxylic fatty acid is behenic acid.

7. A structured oil comprising an oil and a structurant compound as claimed in any one of claims 1 to 6.

8. A structured oil as claimed in claim 7 wherein the oil is one or more of a fatty alcohol polyalkoxylate, an ester oil, a aromatic ester oil; a branched liquid fatty alcohol; a branched liquid fatty acid; a paraffinic oil; and/or a silicone oil.

9. A structured oil as claimed in either claim 7 or claim 8 which contains from 0.1 to 10% by weight of structurant.

10. A personal care product including a structured oil as claimed in any one of claims 7 to 9.

11. A personal care product as claimed in claim 10 in the form of an oil based formulation, a water in oil emulsion or an oil in water emulsion.

12. A personal care product as claimed in either claim 10 or claim 11 which is a lipstick, mascara, cosmetic foundation or cream powder; an antiperspirant and deodorant stick or gel; a baby oil; a hair, massage, make up remover or cleansing product; a shampoo; or a sun care product.

## Patentansprüche

1. Polyesteröl-Strukturbildnerverbindung, die durch Reaktion einer Dimerdicarbonsäure, die sich von Ölsäure, Linolsäure und/oder Linolensäure ableitet, eines Polyols mit mindestens 3 Hydroxylgruppen mit durchschnittlich 1 bis 2 primären Hydroxylgruppen und mindestens einer sekundären Hydroxylgruppe, insbesondere 1 bis 4 sekundären Hydroxylgruppen, und einer C₂₀-C₃₀-Monocarboxylfettsäure, insbesondere einer C₂₀-C₂₄-Monocarboxylfettsäure, erhältlich ist.

2. Polyesteröl-Strukturbildnerverbindung nach Anspruch 1, wobei es sich bei dem Polyol um Glycerin, Sorbitol, Sorbitan oder eine Mischung oder Kombination davon handelt.

3. Polyesteröl-Strukturbildnerverbindung nach Anspruch 1 oder Anspruch 2, wobei die Dimerdicarbonsäure auf Ölsäuredimer basiert.

4. Polyesteröl-Strukturbildnerverbindung nach einem der Ansprüche 1 bis 3, wobei die Dicarbonsäure neben der Dimerdicarbonsäure eine Dicarbonsäure der Formel HOOC-R²-COOH, wobei R² für eine C₂- bis C₈-Hydrocarbylgruppe steht, umfasst.

5. Polyesteröl-Strukturbildnerverbindung nach Anspruch 4, wobei R² für eine Gruppe -(CH₂)ₘ-, wobei m für 2 bis 8 steht, steht.

6. Polyesteröl-Strukturbildnerverbindung nach Anspruch 5, wobei es sich bei der Monocarboxylfettsäure um Behensäure handelt.

7. Strukturiertes Öl, umfassend ein Öl und eine Strukturbildnerverbindung nach einem der Ansprüche 1 bis 6.

8. Strukturiertes Öl nach Anspruch 7, wobei es sich bei dem Öl um ein Fettalkoholpolyalkoxylat, ein Esteröl, ein aromatisches Esteröl, einen verzweigten flüssigen Fettalkohol, eine verzweigte flüssige Fettsäure, ein paraffinisches Öl und/oder ein Silikonöl handelt.

9. Strukturiertes Öl nach Anspruch 7 oder Anspruch 8, das 0,1 bis 10 Gew.-% Strukturbildner enthält.

10. Körperpflegeprodukt mit einem strukturierten Öl nach einem der Ansprüche 7 bis 9.

11. Körperpflegeprodukt nach Anspruch 10 in Form einer ölbasierten Formulierung, einer Wasser-in-Öl-Emulsion oder einer Öl-in-Wasser-Emulsion.

12. Körperpflegeprodukt nach Anspruch 10 oder Anspruch 11, bei dem es sich um einen Lippenstift, eine Wimperntusche, eine kosmetische Grundierung oder ein Cremepuder; einen Antitranspirant- oder Deodorantstift oder ein Antitranspirant- oder Deodorantgel; ein Babyöl; ein Haar-, Massage-, Abschmink- oder Reinigungsprodukt; ein Shampoo oder ein Sonnenschutzprodukt handelt.

## Revendications

1. Composé structurant d'huile de polyester qui est susceptible d'être obtenu par la réaction d'un dimère acide dicarboxylique, issu des acides oléique, linoléique et/ou linolénique, d'un polyol ayant au moins 3 groupements hydroxyle comprenant en moyenne 1 ou 2 groupements hydroxyle primaires et au moins un, en particulier 1 à 4, groupement(s) hydroxyle secondaire(s), et d'un acide gras monocarboxylique en C₂₀ à C₃₀, particulièrement C₂₀ à C₂₄.

2. Composé structurant d'huile de polyester selon la revendication 1, **caractérisé en ce que** le polyol est le glycérol, le sorbitol, le sorbitan ou un mélange ou une combinaison de ceux-ci.

3. Composé structurant d'huile de polyester selon la revendication 1 ou la revendication 2, **caractérisé en ce que** le dimère acide dicarboxylique est à base de dimère d'acide oléique.

4. Composé structurant d'huile de polyester selon l'une quelconque des revendications 1 à 3, **caractérisé en ce que** l'acide dicarboxylique inclut, outre le dimère acide dicarboxylique, un acide dicarboxylique de formule HOOC-R²-COOH, dans laquelle R² est un groupement hydrocarbyle en C₂ à C₈.

5. Composé structurant d'huile de polyester selon la revendication 4, **caractérisé en ce que** R² est un groupement -(CH₂)ₘ-, dans lequel m est de 2 à 8.

6. Composé structurant d'huile de polyester selon la revendication 5, **caractérisé en ce que** l'acide gras monocarboxylique est l'acide béhénique.

7. Huile structurée comprenant une huile et un composé structurant selon l'une quelconque des revendications 1 à 6.

8. Huile structurée selon la revendication 7, **caractérisée en ce que** l'huile est un ou plusieurs parmi un polyalcoxylate d'alcool gras, une huile d'ester, une huile d'ester aromatique ; un alcool gras liquide ramifié ; un acide gras liquide ramifié ; une huile paraffinique ; et/ou une huile de silicone.

9. Huile structurée selon la revendication 7 ou la revendication 8, **caractérisée en ce qu'**elle contient de 0,1 à 10 % en poids de structurant.

10. Produit de soins personnels comportant une huile structurée selon l'une quelconque des revendications 7 à 9.

11. Produit de soins personnels selon la revendication 10, sous forme d'une formulation à base d'huile, d'une émulsion eau dans l'huile ou d'une émulsion huile dans l'eau.

12. Produit de soins personnels selon la revendication 10 ou la revendication 11, **caractérisé en ce qu'**il s'agit d'un rouge à lèvres, mascara, fond de teint cosmétique ou crème poudre ; d'un bâton ou gel antitranspirant et désodorisant ; d'une huile pour bébé ; d'un produit pour les cheveux, de massage, de démaquillage ou nettoyant ; d'un shampooing ; ou d'un produit de protection solaire.
